# EUROPÄISCHE PATENTSCHRIFT

(11) **EP 3 192 482 B1**
(45) Veröffentlichungstag und Bekanntmachung des Hinweises auf die Patenterteilung: **18.12.2019**
(21) Anmeldenummer: 17154688.0
(22) Anmeldetag: 21.06.2013
(51) Int. Cl.: A61F 13/42, A61F 13/45, G01N 27/12

(54) **VORRICHTUNG ZUR ERKENNUNG VON FEUCHTIGKEIT FÜR EINE VORRICHTUNG ZUR ÜBERWACHUNG EINES ZUGANGS ZU EINEM PATIENTEN**
DEVICE FOR DETECTION OF MOISTURE FOR A DEVICE FOR MONITORING ACCESS TO A PATIENT
DISPOSITIF DE RECONNAISSANCE D'HUMIDITÉ POUR UN DISPOSITIF DESTINÉ À SURVEILLER UN ACCÈS À UN PATIENT

(30) Priorität: 09.07.2012 DE 102012013474; 09.07.2012 US 201261669186 P
(43) Veröffentlichungstag der Anmeldung: 19.07.2017
(62) Teilanmeldung aus: 13732358.0
(73) Patentinhaber: Fresenius Medical Care Deutschland GmbH, 61352 Bad Homburg v.d.H. (DE)
(72) Erfinder: Heppe, John, 66606 St. Wendel (DE); Krämer, Andrea, 65933 Frankfurt am Main (DE); Schrörs, Alexander, 60389 Frankfurt am Main (DE); Wüpper, Andreas, 64572 Büttelborn (DE)
(74) Vertreter: Oppermann, Frank

(56) Entgegenhaltungen:
- WO-A1-2011/116943
- WO-A2-2008/021462
- WO-A2-2009/075592

## Beschreibung

Die Erfindung bezieht sich auf eine Vorrichtung zur Erkennung von Feuchtigkeit für eine Vorrichtung zur Überwachung eines Zugangs zu einem Patienten für eine Einrichtung, mit der über eine Schlauchleitung eine Flüssigkeit einem Patienten zugeführt und/oder eine Flüssigkeit von dem Patienten abgeführt wird, insbesondere zur Überwachung des Gefäßzugangs bei einer extrakorporalen Blutbehandlung, insbesondere zur Überwachung eines zentralvenösen Katheters für die akute Dialyse. Darüber hinaus betrifft die Erfindung eine Vorrichtung zur Überwachung eines Zugangs zu einem Patienten, die über eine Vorrichtung zur Erkennung von Feuchtigkeit verfügt.

Auf dem Gebiet der Medizintechnik sind verschiedene Einrichtungen bekannt, mit denen über eine Schlauchleitung Patienten Flüssigkeiten entnommen oder Flüssigkeiten den Patienten zugeführt werden können. Dabei erfolgt der Zugang zu den Patienten im Allgemeinen mit einem Katheter zum Einführen in Körperorgane oder einer Kanüle zum Punktieren von Gefäßen. Während der Untersuchung oder Behandlung ist ein ordnungsgemäßer Zugang zu dem Patienten sicher zu stellen. Daher ist es erforderlich, den Patientenzugang zu überwachen.

Einen ordnungsgemäßen Zugang zu dem Patienten setzen insbesondere auch die extrakorporalen Blutbehandlungsvorrichtungen voraus, die über einen extrakorporalen Blutkreislauf verfügen. Zu den bekannten extrakorporalen Blutbehandlungsvorrichtungen zählen beispielsweise Dialysevorrichtungen und Zellseparatoren, die einen Zugang zu dem Gefäßsystem des Patienten erforderlich machen. Bei der extrakorporalen Blutbehandlung wird dem Patienten über eine arterielle Schlauchleitung mit einer arteriellen Punktionskanüle Blut entnommen, das dem Patienten über eine venöse Schlauchleitung mit einer venösen Punktionskanüle wieder zugeführt wird. Beispielsweise bei der akuten Dialyse auf Intensivstationen wird hingegen ein zentralvenöser Katheter am Hals des Patienten eingesetzt.

Zum Anschluss von Kathetern und Schlauchleitungen für die Herstellung eines Patientenzugangs findet im Allgemeinen das im medizinischen Bereich bekannte Luer-Verbindungssystem Verwendung, dessen Verbindungsteile einen Innen- und einen Außenkonus umfassen. Dieses Verbindungssystem wird als Luer-Lock-Verbindung bezeichnet, wenn der Innen- und Außenkonus zur Sicherung der Verbindung um ein Schraubgewinde erweitert ist. Obwohl die Luer-Lock-Verbindungen eine sehr hohe Sicherheit bieten, hat sich in der Praxis gezeigt, dass sich die Verbindungsteile bei unsachgemäßer Handhabung, bei Materialfehlern oder zu häufigem Gebrauch lösen oder Mikrorisse im Material auftreten können. Die US 2010/0228231 schlägt daher vor, die Verbindungsteile eines Luer-Lock-Verbindungssystems mit einer zusätzlichen Fixierung der Verbindungsteile gegen unbeabsichtigtes Lösen zu sichern.

Zur Überwachung des Gefäßzugangs sind verschiedene Vorrichtungen unterschiedlicher Ausbildung bekannt. Die bekannten Überwachungsvorrichtungen greifen im Allgemeinen auf die standardmäßig in den Blutbehandlungsvorrichtungen vorhandenen Sicherheitsvorrichtungen zurück, die bei einem nicht ordnungsgemäßen Gefäßzugang eine sofortige Unterbrechung des extrakorporalen Blutkreislaufs auslösen.

Es sind Vorrichtungen zur Überwachung eines Gefäßzugangs mit einer Kanüle bekannt, die über eine Vorrichtung zur Erkennung von Feuchtigkeit verfügen, um an der Punktionsstelle das Austreten von Blut erkennen zu können. Die bekannten Vorrichtungen zur Erkennung von Feuchtigkeit, die bei den bekannten Überwachungsvorrichtungen für den Patientenzugang Verwendung finden, sind als Pad ausgebildet, das aus einem saugfähigen Material besteht, in das ein Feuchtigkeitssensor eingebettet ist.

Pads aus einem saugfähigen Material, die auf die Haut des Patienten aufgelegt werden, sind beispielsweise aus der WO 2006/008866 A1, US 2005/0038325 A1 und US 6,445,304 B1 bekannt. Die bekannten Pads zeichnen sich dadurch aus, dass der Feuchtigkeitssensor in das saugfähige Material eingebettet ist und das saugfähige Material auf die Haut des Patienten aufgelegt wird.

Die WO 99/24145 beschreibt eine Vorrichtung zur Erkennung von Feuchtigkeit, die ein mit einem Deckel verschließbares Gehäuse aufweist, in dem ein Feuchtigkeitssensor angeordnet ist. Zur Durchführung der Kanülen und Schlauchleitungen sind in dem Gehäuse Durchbrüche vorgesehen. Nachteilig ist, dass das Gehäuse mit dem Feuchtigkeitssensor in großen Stückzahlen relativ kostspielig herzustellen ist und in der Praxis nur relativ schwierig zu handhaben ist. Weitere Vorrichtungen sind in den Dokumenten WO 2009/075592 A2 und WO 2011/116943 A1 offenbart.

Der Erfindung liegt die Aufgabe zugrunde, eine in großen Stückzahlen kostengünstig herzustellende Vorrichtung zur Erkennung von Feuchtigkeit zu schaffen. Eine weitere Aufgabe der Erfindung ist, eine Vorrichtung zur Erkennung von Feuchtigkeit zu schaffen, die einfach und sicher zu handhaben ist und einen hohen Tragekomfort bietet. Der Erfindung liegt auch die Aufgabe der Erfindung zugrunde, eine Vorrichtung zur Überwachung eines Zugangs zu einem Patienten mit einer derartigen Vorrichtung zur Erkennung von Feuchtigkeit bereitzustellen.

Die Lösung dieser Aufgaben erfolgt erfindungsgemäß mit den Merkmalen der unabhängigen Ansprüche. Die Gegenstände der abhängigen Ansprüche betreffen vorteilhafte Ausführungsformen der Erfindung.

Die erfindungsgemäße Vorrichtung zur Erkennung von Feuchtigkeit weist einen Feuchtigkeitssensor auf, der als eine elektrisch leitende Struktur ausgebildet ist. Der Feuchtigkeitssensor der erfindungsgemäßen Vorrichtung wird an eine Vorrichtung zur Überwachung eines Zugangs zu einem Patienten angeschlossen, die Bestandteil der Einrichtung sein kann, mit der über eine Schlauchleitung eine Flüssigkeit einem Patienten zugeführt und/oder eine Flüssigkeit von dem Patienten abgeführt wird.

Die erfindungsgemäße Vorrichtung zur Erkennung von Feuchtigkeit zeichnet sich dadurch aus, dass zumindest ein Abschnitt der Vorrichtung zur Erkennung von Feuchtigkeit als ein zu einer Manschette verformbarer Abschnitt ausgebildet ist, der um den Umfang der Schlauchleitung oder eines Verbindungssystems der Schlauchleitung gelegt werden kann. Der manschettenförmige Abschnitt weist Befestigungsmittel zur Fixierung des manschettenförmigen Abschnitts in der die Schlauchleitung oder das Verbindungssystem umschließenden Stellung auf.

Der in der Art einer Manschette ausgebildete Abschnitt erlaubt es, die Vorrichtung zur Erkennung von Feuchtigkeit an einer Schlauchleitung oder einem Verbindungssystem der Leitung schnell und sicher zu befestigen. Zusätzliche Befestigungsmittel sind hierzu nicht erforderlich. Beispielsweise ist es nicht erforderlich, die Vorrichtung mit einem Klebeband an der Schlauchleitung, dem Verbindungssystem oder der Haut des Patienten zu fixieren. Dadurch wird die Handhabung der Vorrichtung vereinfacht.

Darüber hinaus ist es von Vorteil, dass sich die erfindungsgemäße Vorrichtung nach der Behandlung wieder leicht von der Schlauchleitung abnehmen lässt, was insbesondere bei der Verwendung zur Überwachung eines zentralvenösen Katheters von Bedeutung ist. Auch während der Behandlung kann die Vorrichtung leicht von der Schlauchleitung gelöst werden.

Die erfindungsgemäße Vorrichtung kann insbesondere in dem Bereich der Schlauchleitung befestigt werden, an dem sich ein Verbindungssystem, beispielsweise ein Luer-Verbindungssystem befindet. Hierzu braucht der zu einer Manschette verformbare Abschnitt nur um das Verbindungssystem gelegt zu werden, so dass sich die Verbindungsstelle auf Leckagen überwachen lässt.

Die erfindungsgemäße Vorrichtung weist vorzugsweise eine dem Patienten zugewandte außenliegende für Flüssigkeit nicht durchlässige Schicht und eine dem Patienten abgewandte innenliegende für Flüssigkeit saugfähige Schicht auf. Dadurch wird erreicht, dass an der Schlauchverbindungsstelle austretendes Blut unmittelbar an den Feuchtigkeitssensor gelangt. Weiterhin wird vermieden, dass Schweiß von der Haut des Patienten oder Blut von einer Undichtigkeit des Blutschlauchsatzes an einer anderen Stelle als dem venösen Anschluss oder von einer Wunde oder sonstige Feuchtigkeit von außen zu dem Feuchtigkeitssensor gelangt, so dass Fehlalarme vermieden werden.

Während eine zusätzliche Sicherung einer Luer-Verbindung, die in der US 2010/0228231 beschreiben ist, nur das Lösen der Verbindung verhindern kann, erlaubt die erfindungsgemäße Vorrichtung auch die Erkennung eines langsamen Blutverlustes, beispielsweise in Folge von Handhabung- oder Materialfehlern der Schlauchverbindung.

Die Befestigungsmittel weisen eine mit einer Klebe- oder Adhäsionsschicht versehene Fläche auf. Vorzugsweise ist die Klebe- oder Adhäsionsfläche ein Streifen, der an dem zu einer Manschette verformbaren Abschnitt vorgesehen ist. Die Klebe- oder Adhäsionsschicht kann auch als Klettverschluss oder eine selbsthaftende Folie ausgebildet sein.

Zur elektrischen Kontaktierung des Feuchtigkeitssensors weist die erfindungsgemäße Vorrichtung Anschlusskontakte auf. Die Anzahl der Anschlusskontakte richtet sich nach der Ausbildung des Feuchtigkeitssensors. Beispielsweise kann der Feuchtigkeitssensor zwei Anschlusskontakte umfassen, zwischen denen der elektrische Widerstand gemessen wird. Für den Anschluss eines Abschlusswiderstandes können noch zwei weitere Anschlusskontakte vorgesehen sein. Anstelle einer Anschlusslasche mit Anschlusskontakten ist es aber auch möglich, eine elektrische Verbindungsleitung aus der erfindungsgemäßen Vorrichtung herauszuführen, die an die Überwachungsvorrichtung angeschlossen wird.

Eine bevorzugte Ausführungsform sieht vor, dass die Anschlusskontakte am Ende eines langgestreckten Abschnitts ausgebildet sind, der sich an den als Manschette ausgebildeten Abschnitt anschließt. Dadurch wird erreicht, dass die Anschlusskontakte räumlich von dem Feuchtigkeitssensor getrennt sind. Daher braucht der Bereich der elektrischen Kontaktierung im Gegensatz zu dem Bereich, an dem sich der Feuchtigkeitssensor befindet, nicht steril zu sein. Dies ist insbesondere bei der Überwachung von zentralvenösen Kathetern von Vorteil.

Bei einer besonders bevorzugten Ausführungsform sind die Anschlusskontakte am Ende des langgestreckten Abschnitts angeordnet, der als Lasche zum Zuziehen des manschettenförmigen Abschnitts dient. Damit kann eine zusätzliche Lasche zur elektrischen Kontaktierung des Feuchtigkeitssensors entfallen.

Die erfindungsgemäße Vorrichtung weist vorzugsweise eine dem Patienten zugewandte außenliegende für Feuchtigkeit nicht durchlässige Schicht und eine dem Patienten abgewandte innenliegende für Flüssigkeit saugfähige Schicht auf. Die elektrisch leitende Struktur des Feuchtigkeitssensors ist vorzugsweise in die für Flüssigkeit saugfähige Schicht eingebettet oder auf die saugfähige Schicht aufgebracht. Die elektrisch leitende Struktur braucht sich nicht über die gesamte Fläche der saugfähigen Schicht erstrecken, da auf Grund der Saugfähigkeit der Schicht die Erkennung von Flüssigkeit in einem Teilbereich der Fläche ausreichend ist.

Bei einer besonders bevorzugten Ausführungsform ist die saugfähige Schicht zur Einbettung der elektrisch leitfähigen Struktur ein textiles Material. Vorzugsweise ist das textile Material ein Gewebe mit nicht-leitfähigen Kettfäden und nicht-leitfähigen Schussfäden sowie leitfähigen Kettfäden und leitfähigen Schussfäden, die derart angeordnet sind, dass die elektrisch leitende Struktur in dem Gewebe ausgebildet ist. Ein derartiges Gewebe mit einer elektrisch leitfähigen Struktur ist in der WO 2011/116943 im Einzelnen beschrieben.

Die elektrisch leitende Struktur kann eine oder mehrere Leiterbahnen aufweisen, die sich in mehreren Abschnitten zumindest über einen Teilbereich des manschettenförmigen Abschnitts erstreckt.

Der zu einer Manschette verformbare Abschnitt kann in seinen Abmessungen und Formen unterschiedlich ausgebildet sein. Bei einer bevorzugten Ausführungsform ist der zu einer Manschette verformbare Abschnitt ein rechteckförmiger Abschnitt, der die Form eines zylindrischen Abschnitts annimmt, der die Schlauchleitung oder das Verbindungssystem umschließt.

Eine alternative Ausführungsform sieht vor, dass der als Manschette verformbare Abschnitt ein kreisförmiger Abschnitt ist, der über einen Teil des Umfangs ausgeschnitten ist. Bei dieser Ausführungsform nimmt der die Schlauchleitung umschließende Abschnitt die Form eines Trichters an. Zur Durchführung der Schlauchleitung weist der über einen Teil seines Umfangs ausgeschnittene Abschnitt im Zentrum einen zentralen, vorzugsweise kreisförmigen Ausschnitt auf.

Die erfindungsgemäße Vorrichtung kann in einem Webprozess ohne großen fertigungstechnischen Aufwand in großen Stückzahlen hergestellt werden. Sie kann weiterhin ohne großen technischen Aufwand ausgeschnitten und sterilisiert und als Einwegsensor in einer geeigneten Verpackung steril bereitgestellt werden.

Die erfindungsgemäße Vorrichtung zur Überwachung eines Zugangs zu einem Patienten, insbesondere zur Überwachung des Gefäßzugangs bei einer extrakorporalen Blutbehandlung, insbesondere zur Überwachung eines zentralvenösen Katheters, verfügt über die erfindungsgemäße Vorrichtung zur Detektion von Feuchtigkeit, die an die Überwachungsvorrichtung angeschlossen wird. Die Überwachungsvorrichtung kann einen akustischen und/oder optischen und/oder taktilen Alarm auslösen, wenn Feuchtigkeit detektiert wird. Auch kann ein Steuersignal für einen Eingriff in die Steuerung der Einrichtung erzeugt werden, mit der eine Schlauchleitung eine Flüssigkeit dem Patienten zugeführt und/oder eine Flüssigkeit von dem Patienten abgeführt wird.

Die erfindungsgemäße Vorrichtung zur Überwachung eines Patientenzugangs kann eine separate Einheit bilden oder Bestandteil der Einrichtung sein, mit der dem Patienten eine Flüssigkeit zugeführt und/oder von dem Patienten Flüssigkeit abgeführt wird, insbesondere Bestandteil der extrakorporalen Blutbehandlungsvorrichtung sein. Wenn die erfindungsgemäße Überwachungsvorrichtung Bestandteil der Blutbehandlungsvorrichtung ist, kann die Überwachungsvorrichtung von bestimmten Baugruppen oder Bauteilen Gebrauch machen, die in der Blutbehandlungsvorrichtung ohnehin vorhanden sind.

Im Folgenden wird ein Ausführungsbeispiel der Erfindung unter Bezugnahme auf die Zeichnungen näher erläutert.

Es zeigen:
- Fig. 1: die wesentlichen Komponenten einer Blutbehandlungsvorrichtung, die über eine Vorrichtung zur Überwachung eines Gefäßzugangs verfügt,
- Fig. 2: eine vereinfachte Darstellung eines ersten Ausführungsbeispiels der erfindungsgemäßen Vorrichtung zur Erkennung von Feuchtigkeit, die an ein Verbindungssystem zum Verbinden eines zentralvenösen Katheters mit einer Schlauchleitung angelegt wird,
- Fig. 3: ein zweites Ausführungsbeispiel der Vorrichtung von Fig. 2 vor dem Anlegen an das Verbindungssystem,
- Fig. 4: ein drittes Ausführungsbeispiel der Vorrichtung in vereinfachter Darstellung,
- Fig. 5: ein viertes Ausführungsbeispiel der Vorrichtung in vereinfachter Darstellung,
- Fig. 6: ein fünftes Ausführungsbeispiel der Vorrichtung in vereinfachter Darstellung,
- Fig. 7: ein sechstes Ausführungsbeispiel der Vorrichtung in vereinfachter Darstellung,
- Fig. 8: ein siebtes Ausführungsbeispiel der Vorrichtung in vereinfachter Darstellung,
- Fig. 9: ein achtes Ausführungsbeispiel der Vorrichtung in vereinfachter Darstellung und
- Fig. 10: ein neuntes Ausführungsbeispiel der Vorrichtung in vereinfachter Darstellung.

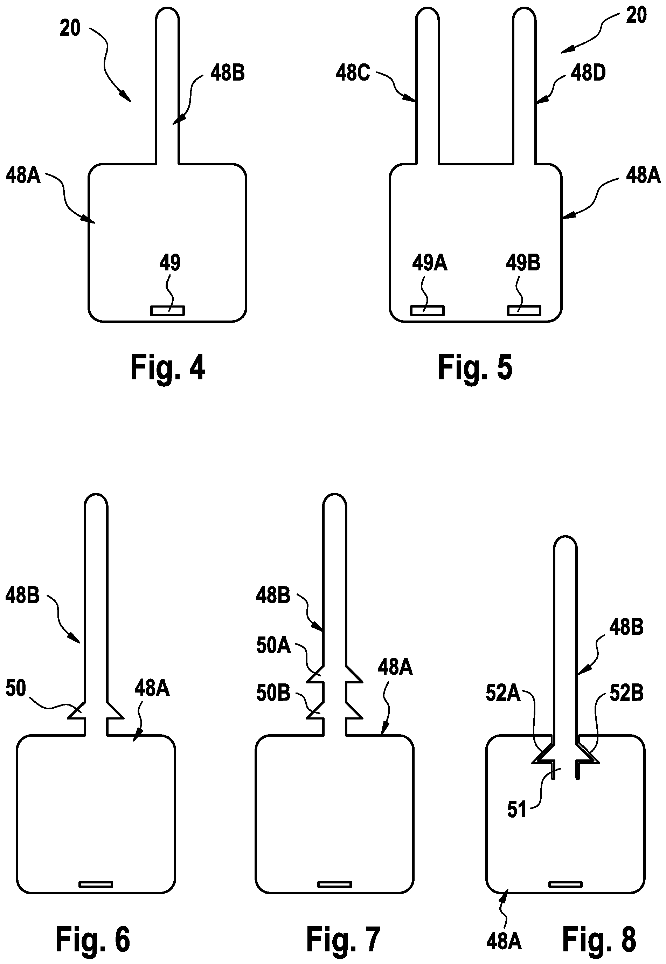

Die in den Figuren 4 bis 10 gezeigten Ausführungsbeispiele, die über Befestigungsmittel verfügen, die einen langgestreckten Abschnitt und einen Schlitz aufweisen, gehören in Bezug auf die Befestigungsmittel nicht zu der Erfindung.

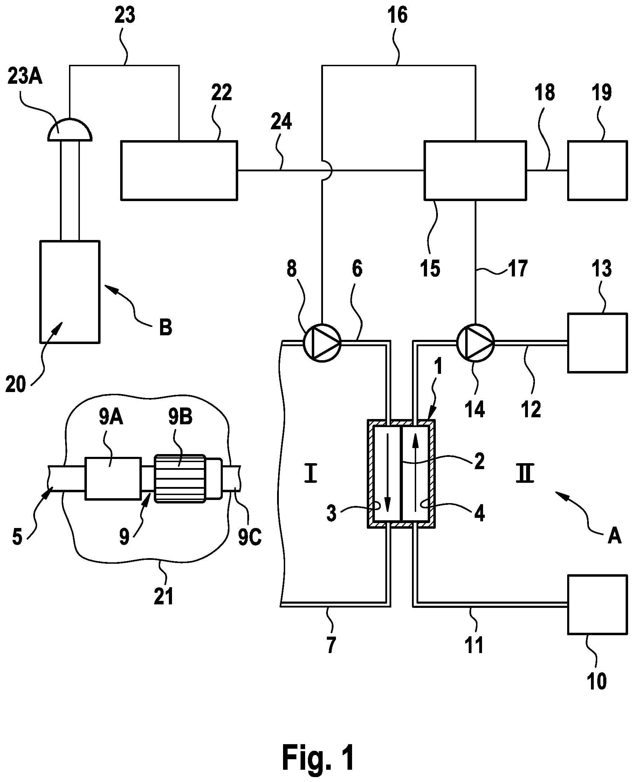

Fig. 1 zeigt die wesentlichen Komponenten einer Blutbehandlungsvorrichtung, insbesondere Hämodialysevorrichtung A für die Akutdialyse, die über eine Vorrichtung B zur Überwachung eines Gefäßzugangs, insbesondere eines Gefäßzugangs mit einem zentralvenösen Katheter verfügt. Bei dem vorliegenden Ausführungsbeispiel ist die Überwachungsvorrichtung B Bestandteil der Hämodialysevorrichtung A. Zunächst wird die Dialysevorrichtung unter Bezugnahme auf Fig. 1 beschrieben.

Die Hämodialysevorrichtung A weist einen Dialysator 1 auf, der durch eine semipermeable Membran 2 in eine Blutkammer 3 und eine Dialysierflüssigkeitskammer 4 unterteilt ist. Der Gefäßzugang zum Patienten erfolgt mit einem zentralvenösen Katheter 5, der am Hals des Patienten angeschlossen wird. Der zentralvenöse Katheter 5 ist Teil des nur andeutungsweise dargestellten extrakorporalen Blutkreislaufs I, der die Blutkammer 3 des Dialysators 1 einschließt und Schlauchleitungen 6, 7 umfasst. Zum Fördern des Bluts im extrakorporalen Kreislauf ist eine Blutpumpe 8 vorgesehen.

Der Dialysierflüssigkeitskreislauf II der Dialysevorrichtung A umfasst eine Dialyseflüssigkeitsquelle 10, an der eine Dialysierflüssigkeitszuführleitung 11 angeschlossen ist, die zu dem Einlass der Dialysierflüssigkeitskammer 4 des Dialysators 1 führt. Von dem Auslass der Dialysierflüssigkeitskammer 4 des Dialysators 1 geht eine Dialysierflüssigkeitsabführleitung 12 ab, die zu einem Auslass 13 führt. In die Dialysierflüssigkeitsabführleitung 12 ist eine Dialysierflüssigkeitspumpe 14 geschaltet.

Die Steuerung der Dialysevorrichtung übernimmt eine zentrale Steuereinheit 15, die über Steuerleitungen 16, 17 die Blut- und Dialysierflüssigkeitspumpe 8, 14 ansteuert. Die zentrale Steuereinheit 15 ist über eine Datenleitung 18 mit einer Alarmeinheit 19 verbunden, die bei einem Störfall einen optischen und/oder akustischen und/oder taktilen Alarm gibt.

Die nur schematisch dargestellte Überwachungsvorrichtung B dient bei dem vorliegenden Ausführungsbeispiel zur Überwachung einer Luer-Lock-Verbindung 9 mit den Verbindungsteilen 9A und 9B zum Anschluss des zentralvenösen Katheters 5 an eine Schlauchleitung 9C des extrakorporalen Blutkreislaufs I. Die Überwachungsvorrichtung B verfügt über eine Vorrichtung 20 zur Erkennung von Feuchtigkeit, die an der Schlauchverbindungsstelle 21 angeordnet wird. Diese Vorrichtung 20 ist in Fig. 1 nur schematisch dargestellt. Darüber hinaus verfügt die Überwachungsvorrichtung B über eine Auswerteinrichtung 22, die über eine Verbindungsleitung 23 mit der Vorrichtung 20 zur Erkennung von Feuchtigkeit elektrisch verbunden ist. Die Verbindungsleitung 23 ist mit einem elektrischen Verbindungsstück 23A an die Vorrichtung 20 angeschlossen.

Über eine Datenleitung 24 ist die Auswerteinrichtung 22 mit der zentralen Steuereinheit 15 der Dialysevorrichtung A verbunden. Für den Fall, dass Blut aus der Schlauchverbindungsstelle 21 austritt und die Vorrichtung 20 zur Erkennung von Feuchtigkeit befeuchtet, erzeugt die Auswerteinrichtung 22 der Überwachungsvorrichtung B ein Steuersignal, das die zentrale Steuereinheit 15 über die Datenleitung 24 empfängt, die einen Eingriff in die Blutbehandlung vornimmt. Die Steuereinheit 15 stoppt die Blutpumpe 8 und erzeugt ein Alarmsignal, sodass die Alarmeinheit 19 einen akustischen und/oder optischen und/oder taktilen Alarm gibt.

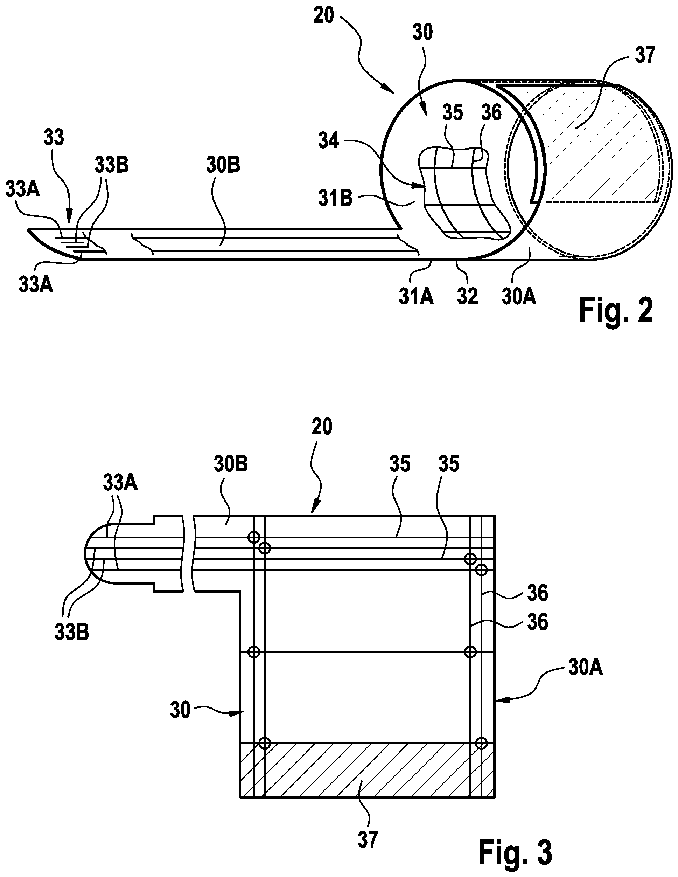

Die Fig. 2 zeigt ein erstes Ausführungsbeispiel der erfindungsgemäßen Vorrichtung zur Erkennung von Feuchtigkeit. Die Vorrichtung weist ein textiles Trägermaterial 30 auf, das bei dem Ausführungsbeispiel ein Gewebe ist. Das Gewebe weist einen Abschnitt 30A mit einem rechteckförmigen Zuschnitt auf, der um den Umfang einer Schlauchleitung oder eines Verbindungssystems zum Verbinden eines Katheters mit einer Schlauchleitung gelegt werden kann. An einer der beiden Längsseiten des rechteckförmigen Abschnitts 30A schließt sich mittig zwischen den beiden Schmalseiten ein langgestreckter Abschnitt 30B an, der eine Anschlusslasche für ein nicht dargestelltes Verbindungsstück zum Anschluss der erfindungsgemäßen Vorrichtung an die Auswerteinrichtung 22 der Vorrichtung B zur Überwachung des Gefäßzugangs bildet.

Das saugfähige Gewebe 30 weist eine der Haut des Patienten zugewandte Außenseite 31A und eine der Haut des Patienten abgewandte Innenseite 31B auf. An der Außenseite 31A ist das Gewebe 30 vorzugsweise vollflächig mit einer Kunststofffolie 32 verbunden, die für Flüssigkeit nicht durchlässig und elektrisch nicht leitend ist. Darüber hinaus verleiht die Kunststofffolie 32 dem Gewebe 30 insbesondere im Bereich des langgestreckten Abschnitts 30B zum Anschluss des Anschlussstücks die erforderliche Festigkeit und/oder Steifigkeit. Als Alternative zur Verwendung einer Kunststofffolie kann eine einseitige hydrophobe Beschichtung des Gewebes vorgesehen werden. Eine Hydrophobisierung eines PES (Polyster)-Gewebes kann beispielsweise durch Einsatz von fluorhaltigen Gasen in einem Niederdruckplasma erfolgen.

Die Vorrichtung zur Erkennung von Feuchtigkeit weist einen in Fig. 1 nur andeutungsweise dargestellten Feuchtigkeitssensor 34 auf, der aus einer elektrisch leitenden Struktur besteht, die in das Gewebe 30 eingebettet ist (Fig. 2). Das Gewebe 30 besteht hierzu aus nicht-leitfähigen Kettfäden und nicht-leitfähigen Schussfäden sowie leitfähigen Kettfäden und leitfähigen Schussfäden, die derart angeordnet sind, dass sich die elektrisch leitende Struktur ergibt, die sich aus einer Mehrzahl von orthogonal zueinander stehenden nur andeutungsweise dargestellten Leiterbahnabschnitten 35, 36 zusammensetzt. Die einzelnen Leiterbahnabschnitte 35, 36 erstrecken sich über Teilbereiche des als Manschette formbaren rechteckförmigen Abschnitts 30A und führen bis an das Ende des langgestreckten Abschnitts 30B zum Anschluss des nicht dargestellten Verbindungsstücks, wobei die Leiterbahnabschnitte zwei Elektroden ausbilden. Am Ende des langgestreckten Abschnitts 30B sind die Leiterbahnabschnitte als Anschlusskontakte 33 ausgebildet. Die äußeren Anschlusskontakte 33A dienen dem Anschluss für die Widerstandsmessung des Feuchtigkeitssensors, während die inneren Anschlusskontakte 33B dem Anschluss eines Abschlusswiderstandes dienen.

An einer Schmalseite weist der zu einer Manschette verformbare Abschnitt 30A eine streifenförmige Klebe- oder Adhäsionsschicht 37 auf, die in Fig. 1 durch eine schraffierte Fläche gekennzeichnet ist. Die Klebe- oder Adhäsionsschicht 37 kann auch an beiden Schmalseiten des Abschnitts 30A vorgesehen sein. Sie kann an der Innen- oder Außenseite des Abschnitts 30A vorgesehen sein. Entscheidend ist, dass sich der Abschnitt 30A um den Umfang des Verbindungssystems oder der Schlauchleitung legen und die einander überlappenden Abschnitte sich miteinander verbinden lassen, wenn der manschettenförmige Abschnitt 30A das Verbindungssystem oder die Schlauchleitung eng umschließt. Die Klebe- oder Adhäsionsschicht 37 kann beispielsweise eine selbsthaftende Folie oder ein Klettverschluss sein. Beispielsweise kann die auf die Außenseite des Gewebes aufgebrachte Kunststofffolie im Randbereich als Klebefolie ausgebildet sein.

Durch Lösen der Klebe- oder Adhäsionsverbindung kann die Vorrichtung zur Erkennung von Feuchtigkeit wieder leicht von der Schlauchleitung oder dem Verbindungssystem abgenommen werden, so dass das Verbindungssystem wieder zugänglich ist.

Wenn die Vorrichtung zur Erkennung von Feuchtigkeit an die Schlauchleitung oder das Verbindungssystem angelegt ist, führt eine Leckage dazu, dass das Gewebe 30 mit Feuchtigkeit, beispielsweise Blut, benetzt wird, wodurch sich das Gewebe mit der Flüssigkeit vollsaugt. Dadurch verändert sich der elektrische Widerstand zwischen den äußeren Anschlusskontakten 33A der elektrischen leitenden Struktur. Der Sensor ist überall dort sensitiv, wo leitfähige Fäden der ersten Elektrode durch Feuchtigkeit mit leitfähigen Fäden der zweiten Elektrode kurzgeschlossen werden können.

Fig. 3 zeigt ein zweites Ausführungsbeispiel der erfindungsgemäßen Vorrichtung 20 vor dem Anlegen an die Schlauchleitung bzw. dessen Verbindungssystem, d. h. wenn die Vorrichtung noch nicht die Form einer Manschette bildet. Dieses Ausführungsbeispiel unterscheidet sich von der ersten Ausführungsform nur dadurch, dass sich der langestreckte Abschnitt 30B nicht mittig, sondern seitlich an den rechteckförmigen Abschnitt 30A anschließt. Die einander entsprechenden Teile sind daher mit den gleichen Bezugszeichen versehen. Fig. 3 zeigt deutlich die orthogonal zueinander stehenden Leiterbahnabschnitte 35, 36. Die sich über Teilbereiche des als Manschette formbaren rechteckförmigen Abschnitts 30A erstreckenden Leiterbahnabschnitte 35, 36 führen bis an das Ende des langgestreckten Abschnitts 30B zum Anschluss des nicht dargestellten Verbindungsstücks. Die Kreuzungspunkte, an denen die orthogonal zueinander verlaufenden Kett- und Schussfäden in dem Gewebe elektrisch kontaktieren, sind mit einem Kreis gekennzeichnet. Ein derartiges Gewebe mit einer elektrisch leitenden Struktur ist in der WO 2011/116943 im Einzelnen beschrieben, auf die ausdrücklich Bezug genommen wird.

Die Figuren 4 bis 10 zeigen alternative Ausführungsformen der Vorrichtung zur Erkennung von Feuchtigkeit, die sich von den unter Bezugnahme auf die Figuren 2 und 3 beschriebenen Ausführungsbeispielen im Wesentlichen durch die Ausbildung der Befestigungsmittel unterscheiden. In Bezug auf die Befestigungsmittel, die einen langgestreckten Abschnitt und einen Schlitz aufweisen, gehören diese Ausführungsformen der Vorrichtung aber nicht zu der Erfindung.

Bei den Ausführungsbeispielen der Figuren 4 bis 8 sind nur die Umrisslinien dargestellt. Die elektrisch leitende Struktur, die sich an der Innenseite des Gewebes befindet, ist nicht dargestellt, da sich die Ausführungsbeispiele der Figuren 4 bis 8 von den Ausführungsformen der Figuren 2 und 3 im Wesentlichen nur durch die Befestigungsmittel und die unterschiedliche Form unterscheiden.

Bei dem Ausführungsbeispiel von Fig. 4 ist der als Manschette verformbare Abschnitt 48A ein im Wesentlichen rechteckförmiger Abschnitt. An eine der Seiten schließt sich an den rechteckförmigen Abschnitt 48A mittig ein langgestreckter Abschnitt 48B an, der wieder eine Anschlusslasche für das nicht dargestellte Verbindungsstück zum Anschluss der Vorrichtung zur Erkennung von Feuchtigkeit an die Auswerteinrichtung der Überwachungsvorrichtung bildet. An der Seite, die dem langgestreckten Abschnitt 48B gegenüberliegt, weist der rechteckförmige Abschnitt 48A einen Schlitz 49 auf, dessen Breite geringfügig größer als die Breite des langgestreckten Abschnitts 48B ist. Wenn der Abschnitt 48A um die Schlauchleitung oder das Verbindungssystem gelegt wird, kann die Manschette festgezogen werden, indem der langgestreckte Abschnitt 48B durch den Schlitz 49 gezogen und anschließend fixiert wird. Auch bei diesem Ausführungsbeispiel dient der langgestreckte Abschnitt also nicht nur der Fixierung der Manschette, sondern auch dem elektrischen Anschluss des Feuchtigkeitssensors.

Fig. 5 zeigt ein alternatives Ausführungsbeispiel, dass sich von dem Ausführungsbeispiel von Fig. 4 nur dadurch unterscheidet, dass anstelle eines mittigen langgestreckten Abschnitts an einer Seite des rechteckförmigen Abschnitts 48A zwei außermittig angeordnete langgestreckte Abschnitte 48C und 48D vorgesehen sind. Die einander entsprechenden Teile sind wieder mit den gleichen Bezugszeichen versehen. Bei dem Ausführungsbeispiel von Fig. 5 ist nur eine der beiden langgestreckten Abschnitte, beispielsweise der linke Abschnitt 48C, als Anschlusslasche für das Anschlussstück ausgebildet. An der den langgestreckten Abschnitten 48C und 48D gegenüberliegenden Seite weist der rechteckförmige Abschnitt 48A zwei außermittig angeordnete Schlitze 49A und 49B auf, durch die die langgestreckten Abschnitte 48C, 48D gezogen werden, um die Manschette festzuziehen.

Die Fig. 6 bis 8 zeigen alternative Ausführungsformen, die sich von dem Ausführungsbeispiel von Fig. 4 nur durch die besondere Form des langgestreckten Abschnitts 48B unterscheiden. Während bei dem Ausführungsbeispiel der Fig. 4 der langgestreckte Abschnitt mit zusätzlichen Fixierungsmitteln fixiert werden muss, verfügt der langgestreckte Abschnitt 48B der Fig. 6 über einen Widerhaken 50, der verhindert, dass der langgestreckte Abschnitt 48B wieder aus dem Schlitz 49 herausgezogen werden kann. Fig. 7 zeigt ein Ausführungsbeispiel mit zwei im Abstand zueinander angeordneten Widerhaken 50A und 50B. Es können aber auch noch weitere Widerhaken vorgesehen sein, um die Manschette auf den gewünschten Durchmesser zusammenziehen zu können.

Fig. 8 zeigt eine weitere Ausführungsform, die sich von den oben beschriebenen Ausführungsbeispielen nur dadurch unterscheidet, dass der Widerhaken 51 in den rechteckförmigen Abschnitt 48A integriert ist. Der sich an den rechteckförmigen Abschnitt 48A anschließende langgestreckte Abschnitt 48B bildet in dem Bereich, an dem der langgestreckte Abschnitt 48B an den rechteckförmigen Abschnitt 48A angeschlossen ist, einen Ausschnitt des rechteckförmigen Abschnitts, wobei die Außenkontur an den Längsseiten des langgestreckten Abschnitts 48B Einschnitte 52A, 52B in dem rechteckförmigen Abschnitt 48A sind. Dadurch lässt sich der langgestreckte Abschnitt 48B aus dem rechteckförmigen Abschnitt 48A herausklappen, so dass der Widerhaken 52 freiliegt. Auf diese Weise können auch mehrere Widerhaken ausgebildet sein.

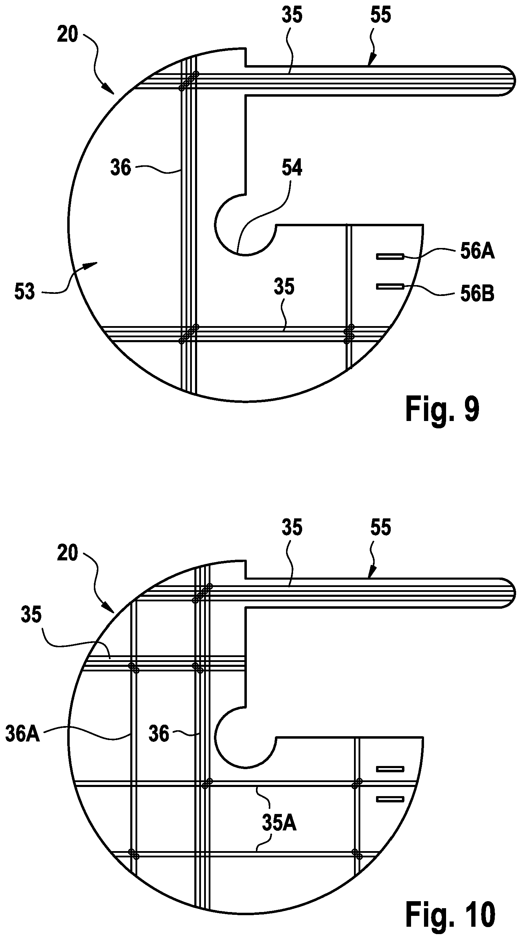

Fig. 9 zeigt ein Ausführungsbeispiel, bei dem der zu einer Manschette formbare Abschnitt 53 ein kreisförmiger Abschnitt ist, der über einen Teil des Umfangs ausgeschnitten ist. Bei dem vorliegenden Ausführungsbeispiel ist der kreisförmige Abschnitt 53 über einen Umfang von 90 Grad ausgeschnitten. Im Zentrum weist der ausgeschnittene kreisförmige Abschnitt 53 einen zentralen kreisförmigen Ausschnitt 54 auf, dessen Durchmesser größer als der Durchmesser der Schlauchleitung oder des Verbindungssystems ist. An der einen Hälfte des ausgeschnittenen kreisförmigen Abschnitts 53 schließt sich ein langgestreckter Abschnitt 55 an, der wieder die Anschlusslasche für das elektrische Anschlussstück bildet. Auf der anderen Hälfte des Abschnitts 53 befinden sich zwei im Abstand zueinander angeordnet Schlitze 56A und 56B, durch die der langgestreckte Abschnitt 55 gezogen werden kann. Bei diesem Ausführungsbeispiel kann der langgestreckte Abschnitt 55 dadurch fixiert werden, dass er durch beide Schlitze von oben bzw. von unten gezogen wird. Zum Anlegen der Vorrichtung 20 zur Erkennung von Feuchtigkeit wird der ausgeschnittene kreisförmige Abschnitt 53 um die Schlauchleitung oder das Verbindungssystem gelegt, wobei die Schlauchleitung oder das Verbindungssystem in dem zentralen Ausschnitt 54 zum Liegen kommt. Anschließend wird der ausgeschnittene kreisförmige Abschnitt 53 zu einer Manschette in der Form eines Trichters geformt, der die Schlauchleitung oder das Verbindungssystem umschließt. Die Fixierung erfolgt dann mit der Anschlusslasche 55. Die Leiterbahnabschnitte 35, 36 der elektrisch leitenden Struktur 34, die den Feuchtigkeitssensor bildet, ist in Fig. 9 dargestellt.

Fig. 10 zeigt ein alternatives Ausführungsbeispiel, das sich von der Ausführungsform der Fig. 9 nur durch die Anordnung der Leiterbahnabschnitte 35, 36 der elektrisch leitenden Struktur 34 unterscheidet. Bei dem Ausführungsbeispiel von Fig. 10 verfügt die elektrisch leitende Struktur 34 neben den Leiterbahnabschnitten 35, 36 noch über zusätzliche Leiterbahnabschnitte 35A, 36A, so dass der Feuchtigkeitssensor von Fig. 10 eine höhere Sensitivität als der Sensor von Fig. 9 aufweist.

## Patentansprüche

1. Vorrichtung zur Erkennung von Feuchtigkeit für eine Vorrichtung zur Überwachung eines Zugangs zu einem Patienten für eine Einrichtung, mit der über eine Schlauchleitung eine Flüssigkeit einem Patienten zugeführt und/oder eine Flüssigkeit von dem Patienten abgeführt wird, insbesondere zur Überwachung des Gefäßzugangs bei einer extrakorporalen Blutbehandlung,
wobei die Vorrichtung zur Erkennung von Feuchtigkeit einen Feuchtigkeitssensor (34) aufweist, der als eine elektrisch leitende Struktur ausgebildet ist, wobei zumindest ein Abschnitt der Vorrichtung zur Erkennung von Feuchtigkeit als ein zu einer Manschette verformbarer Abschnitt (30A) ausgebildet ist, der um den Umfang einer Schlauchleitung oder eines Verbindungsystems einer Schlauchleitung gelegt werden kann, wobei die Vorrichtung zur Erkennung von Feuchtigkeit Befestigungsmittel (37, 48, 55) zur Fixierung des manschettenförmigen Abschnitts (30A) in der die Schlauchleitung oder das Verbindungssystem umschließenden Stellung aufweist,
**dadurch gekennzeichnet, dass**
die Befestigungsmittel des zu einer Manschette verformbaren Abschnitts (30A) eine mit einer Klebe- oder Adhäsionsschicht (37) versehene Fläche aufweisen, und
die Vorrichtung zur Erkennung von Feuchtigkeit Anschlusskontakte (33) zur elektrischen Kontaktierung des Feuchtigkeitssensors (34) aufweist, wobei die Anschlusskontakte (33) am Ende eines langgestreckten Abschnitts (30B) ausgebildet sind, der sich an den als Manschette verformbaren Abschnitt (30A) anschließt.

2. Vorrichtung nach Anspruch 1, **dadurch gekennzeichnet, dass** die Vorrichtung zur Erkennung von Feuchtigkeit eine dem Patienten zugewandte außenliegende für Feuchtigkeit nicht durchlässige Schicht (31A) und eine dem Patienten abgewandte innenliegende für Flüssigkeit saugfähige Schicht (31B) aufweist.

3. Vorrichtung nach Anspruch 2, **dadurch gekennzeichnet, dass** die innenliegende für Flüssigkeit saugfähige Schicht ein textiles Material (30) ist, das die elektrisch leitende Struktur (34) aufweist.

4. Vorrichtung nach einem der Ansprüche 1 bis 3, **dadurch gekennzeichnet, dass** die elektrisch leitende Struktur (34) in das textile Material (30) eingebettet ist.

5. Vorrichtung nach Anspruch 4, **dadurch gekennzeichnet, dass** das textile Material ein Gewebe (30) mit nicht-leitfähigen Kettfäden und nicht-leitfähigen Schussfäden sowie leitfähigen Kettfäden und leitfähigen Schussfäden ist, die derart angeordnet sind, dass die elektrisch leitende Struktur (34) in dem Gewebe (30) ausgebildet ist.

6. Vorrichtung nach einem der Ansprüche 1 bis 5, **dadurch gekennzeichnet, dass** die elektrisch leitende Struktur (34) mindestens eine Leiterbahn (35, 36) aufweist, die sich in mehreren Abschnitten zumindest über einen Teilbereich des manschettenförmigen Abschnitts (30A) erstreckt.

7. Vorrichtung nach einem der Ansprüche 1 bis 6, **dadurch gekennzeichnet, dass** der zu einer Manschette verformbare Abschnitt (30A) ein rechteckförmiger Abschnitt ist.

8. Vorrichtung nach einem der Ansprüche 1 bis 7, **dadurch gekennzeichnet, dass** der zu einer Manschette verformbare Abschnitt (53) ein kreisförmiger Abschnitt ist, der über einen Teil des Umfangs ausgeschnitten ist.

9. Vorrichtung nach Anspruch 8, **dadurch gekennzeichnet, dass** der kreisförmige Abschnitt (53), der über einen Teil des Umfangs ausgeschnitten ist, im Zentrum einen zentralen Ausschnitt (54) aufweist.

10. Vorrichtung nach einem der Ansprüche 1 bis 9, **dadurch gekennzeichnet, dass** die Befestigungsmittel des zu einer Manschette verformbaren Abschnitts (30A) einen mit einer Klebe- oder Adhäsionsschicht versehenen Streifen aufweisen.

11. Vorrichtung nach einem der Ansprüche 1 bis 10, **dadurch gekennzeichnet, dass** die Klebe- oder Adhäsionsschicht (37) als Klettverschluss oder als eine selbsthaftende Folie ausgebildet ist.

12. Vorrichtung zur Überwachung eines Zugangs zu einem Patienten für eine Einrichtung, mit der über eine Schlauchleitung eine Flüssigkeit einem Patienten zugeführt und/oder eine Flüssigkeit von dem Patienten abgeführt wird, insbesondere zur Überwachung des Gefäßzugangs bei einer extrakorporalen Blutbehandlung, **dadurch gekennzeichnet, dass** die Überwachungsvorrichtung eine Vorrichtung zur Erkennung von Feuchtigkeit (20) nach einem der Ansprüche 1 bis 11 aufweist.

## Claims

1. Device for detecting moisture for an arrangement for monitoring an access to a patient for a system by which, via a flexible line, a liquid is fed to a patient and/or a liquid is fed out from the patient, and in particular for monitoring the vascular access in extra-corporeal blood treatment, the device for detecting moisture having a moisture sensor (34) which takes the form of an electrically conductive structure, at least a portion of the device for detecting moisture taking the form of a portion (30A) which can be deformed into a sleeve and which can be placed around the circumference of a flexible line or of a system for connecting a flexible line, the device for detecting moisture having fastening means (37, 48, 55) for fixing the portion (30A) in sleeve form in place in the position in which it surrounds the flexible line or the connecting system,
**characterised in that** the fastening means of the portion (30A) able to be deformed into a sleeve have an area which is provided with an adhesive or adhering layer (37), and
the device for detecting moisture has connecting contacts (33) to allow electrical contact to be made with the moisture sensor (34), the connecting contacts (33) being formed at the end of an elongated portion (30B) which is connected to the portion (30A) able to be formed into a sleeve.

2. Device according to claim 1, **characterised in that** the device for detecting moisture has a layer (31A) not permeable to moisture which is adjacent the patient and situated on the outside and a layer (31B) absorbent of liquid which is remote from the patient and situated on the inside.

3. Device according to claim 2, **characterised in that** layer absorbent of liquid which is situated on the inside is a textile material (30) which has the electrically conductive structure (34).

4. Device according to one of claims 1 to 3, **characterised in that** the electrically conductive structure (34) is embedded in the textile material (30).

5. Device according to claim 4, **characterised in that** the textile material is a woven material (30) having non-conductive warp filaments and non-conductive weft filaments and conductive warp filaments and conductive weft filaments, which are so arranged that the electrically conductive structure (34) is formed in the woven material (30).

6. Device according to one of claims 1 to 5, **characterised in that** the electrically conductive structure (34) has at least one conductor (35, 36) which, in a plurality of portions, extends at least over a sub-region of the portion (30A) in sleeve form.

7. Device according to one of claims 1 to 6, **characterised in that** the portion (30A) able to be deformed into a sleeve is a square-cornered portion.

8. Device according to one of claims 1 to 7, **characterised in that** the portion (53) able to be deformed into a sleeve is a circular portion which is cut away for part of its circumference.

9. Device according to claim 8, **characterised in that** the circular portion (53) which is cut away for part of its circumference has in the centre a central cut-out (54).

10. Device according to one of claims 1 to 9, **characterised in that** the fastening means of the portion (30A) able to be deformed into a sleeve have a strip which is provided with an adhesive or adhering layer.

11. Device according to one of claims 1 to 10, **characterised in that** the adhesive or adhering layer (37) is formed as a hook-and-loop fastener or a self-adhesive film.

12. Arrangement for monitoring an access to a patient for a system by which, via a flexible line, a liquid is fed to a patient and/or a liquid is fed out from the patient, and in particular for monitoring the vascular access in extra-corporeal blood treatment, **characterised in that** the monitoring arrangement has a device (20) for detecting moisture according to one of claims 1 to 11.

## Revendications

1. Dispositif de détection d'humidité pour un dispositif de surveillance d'un accès à un patient pour un équipement avec lequel, par le biais d'une conduite tubulaire, un liquide est acheminé à un patient et/ou un liquide est évacué du patient, en particulier pour la surveillance de l'accès vasculaire dans le cas d'un traitement extracorporel du sang,
le dispositif de détection d'humidité comportant un capteur d'humidité (34) qui est constitué en tant que structure électriquement conductrice, au moins un segment du dispositif de détection d'humidité étant constitué en tant que segment (30A) déformable en forme de manchette qui peut être placé autour de la circonférence d'une conduite tubulaire ou d'un système de raccordement d'une conduite tubulaire, le dispositif de détection d'humidité comportant des moyens de fixation (37, 48, 55) pour l'immobilisation du segment (30A) en forme de manchette dans la position entourant la conduite tubulaire ou le système de raccordement, **caractérisé en ce que**
les moyens de fixation du segment (30A) déformable en forme de manchette comportent une surface munie d'une couche de colle ou d'adhésif (37), et
le dispositif de détection d'humidité comporte des contacts de connexion (33) pour la mise en contact électrique du capteur d'humidité (34), les contacts de connexion (33) étant constitués à l'extrémité d'un segment (30B) allongé qui se raccorde au segment (30A) déformable en forme de manchette.

2. Dispositif selon la revendication 1, **caractérisé en ce que** le dispositif de détection d'humidité comporte une couche (31A) extérieure imperméable à l'humidité tournée vers le patient et une couche (31B) intérieure absorbant le liquide éloignée du patient.

3. Dispositif selon la revendication 2, **caractérisé en ce que** la couche intérieure absorbant le liquide est un matériau (30) textile qui comporte la structure (34) électriquement conductrice.

4. Dispositif selon l'une des revendications 1 à 3, **caractérisé en ce que** la structure (34) électriquement conductrice est noyée dans le matériau (30) textile.

5. Dispositif selon la revendication 4, **caractérisé en ce que** le matériau textile est un tissu (30) avec des fils de chaîne non conducteurs et des fils de trame non conducteurs, ainsi qu'avec des fils de chaîne conducteurs et des fils de trame conducteurs qui sont disposés de telle sorte que la structure (34) électriquement conductrice est constituée dans le tissu (30).

6. Dispositif selon l'une des revendications 1 à 5, **caractérisé en ce que** la structure (34) électriquement conductrice comporte au moins une piste conductrice (35, 36) qui s'étend dans plusieurs segments au moins sur une zone partielle du segment (30A) en forme de manchette.

7. Dispositif selon l'une des revendications 1 à 6, **caractérisé en ce que** le segment (30A) déformable en forme de manchette est un segment de forme rectangulaire.

8. Dispositif selon l'une des revendications 1 à 7, **caractérisé en ce que** le segment (53) déformable en forme de manchette est un segment de forme circulaire qui est découpé sur une partie de la circonférence.

9. Dispositif selon la revendication 8, **caractérisé en ce que** le segment (53) de forme circulaire qui est découpé sur une partie de la circonférence comporte au centre une découpe (54) centrale.

10. Dispositif selon l'une des revendications 1 à 9, **caractérisé en ce que** les moyens de fixation du segment (30A) déformable en forme de manchette comportent une bande munie d'une couche de colle ou d'adhésif.

11. Dispositif selon l'une des revendications 1 à 10, **caractérisé en ce que** la couche de colle ou d'adhésif (37) est constituée en tant que fermeture auto-agrippante ou en tant que feuille auto-adhésive.

12. Dispositif de surveillance d'un accès à un patient pour un équipement avec lequel, par le biais d'une conduite tubulaire, un liquide est acheminé à un patient et/ou un liquide est évacué du patient, en particulier pour la surveillance de l'accès vasculaire dans le cas d'un traitement extracorporel du sang, **caractérisé en ce que** le dispositif de surveillance comporte un dispositif de détection d'humidité (20) selon l'une des revendications 1 à 11.
